Europäisches Patentamt

European Patent Office

Office européen des brevets

(11) Publication number: **0 237 507 B1**

# EUROPEAN PATENT SPECIFICATION

(45) Date of publication of patent specification: **27.12.91**  (51) Int. Cl.⁵: **A61M 15/00**

(21) Application number: **87850060.2**

(22) Date of filing: **20.02.87**

(54) Device in powder inhalators.

(30) Priority: **07.03.86 SE 8601060**

(43) Date of publication of application:
**16.09.87 Bulletin 87/38**

(45) Publication of the grant of the patent:
**27.12.91 Bulletin 91/52**

(84) Designated Contracting States:
**AT BE CH DE ES FR GB GR IT LI LU NL SE**

(56) References cited:
**EP-A- 0 052 284**
**US-A- 2 604 094**
**US-A- 2 674 999**
**US-A- 4 524 769**

(73) Proprietor: **Aktiebolaget Draco**
**Box 1707 Tunavägen 43**
**S-221 01 Lund(SE)**

(72) Inventor: **Wetterlin, Kjell Ingvat Leopold**
**Västervang 19**
**S-240 17 S Sandby(SE)**
Inventor: **Virtanen, Risto**
**Torenvägen 14C**
**SF-01900 Nurmijärvi(FI)**
Inventor: **Andersson, Jan Anders Roland**
**Lundavägen 5**
**S-240 17 S Sandby(SE)**

(74) Representative: **Hjertman, Ivan T. et al**
**AB ASTRA Patent and Trade Mark Department**
**S-151 85 Södertälje(SE)**

Rank Xerox (UK) Business Services

## Description

The present invention relates to a powder inhalators according to the preamble of claim 1 intended to be used for local administration of drugs to the respiratory tract and lungs of a patient. Such a device is disclosed in US-A-4 524 769.

There are different types of powder inhalators, for example those which operate with hard gelatine capsules from which the pharmacologically active compound is released during inhalation through the inhalator, and those which dose the pharmacologically active compound directly into the air conduit by means of a special dosing unit, said compound being administered to patients during inhalation through the inhalator.

All substances which are used in such inhalators are atomized or micronized so that the main fraction of the substance is within the particle size range which is termed the respirable range, that is, particles smaller than 5 $\mu$m. This applies both to inhalators operating with pure active compound and to those where the active compound is mixed with suitable diluting agents such as lactose, sucrose etc.

The active compound is enclosed, without carrier substance or when appropriate together with carrier substance, in hard gelatine capsules or directly in a storage unit in the inhalator, which storage unit is connected to a suitable dosing unit incorporated in the powder inhalator. When the substance is to be released from the hard gelatine capsule or from the dosing unit into the air conduit of the powder inhalator, it is essential that the largest possible quantity of primary particles is obtained in the respirable range, that is, smaller than 5 $\mu$m, at flows which can be generated by a patient suffering from disease of the respiratory tract. A sufficient quantity of particles smaller than 5 $\mu$m can be obtained to achieve a therapeutic effect by means of a particle disintegrating construction according to US-A-4 524 769, in which a constriction in the nozzle unit increases the flow velocity of the inhalation air and a propeller contributes to an increase in the quantity of particles in the respirable range. This construction implies, however, that movable parts are used in the nozzle unit.

The object of the invention is to accomplish, against this background, a device in powder inhalators of the known kind which is stated in the preamble of claim 1, in such way that during inhalation and without the help of movable parts an effective disintegration of powder aggregates into particles within the respirable range is achieved.

This object is attained in that the inhalator is provided with the features set forth in the characterizing portion of claim 1.

During the deflection the particles will on the one hand be dashed against the walls of the deflector devices by centrifugal force, whereby large particles or particle aggregates are ground into small particles, and on the other hand collide with each other which results in a mutual grinding action between the particles. The overall result is in that a great quantity of particles can be generated within the respiratory range.

The deflector devices can be arranged in many different ways, in particular in the form of helical channel portions, as will be evident from the claims and the detailed description below.

The invention is described in greater detail below with reference to the accompanying drawings which illustrate some working examples.

Figure 1 shows an axial cross-section through a first embodiment of a device according to the invention with a helical channel portion in the nozzle unit of the powder inhalator;

Figure 2 shows a corresponding view of a second embodiment with a helical channel portion in the container unit of the powder inhalator;

Figure 3 shows a corresponding view of a third embodiment with a helical channel portion in both the nozzle and the container unit;

Figure 4 shows a corresponding view of a fourth embodiment with double helical channel portions in the container unit, and

Figure 5 shows a corresponding view of a fifth embodiment with double helical channel portions in the container unit and with a helical channel portion in the nozzle unit.

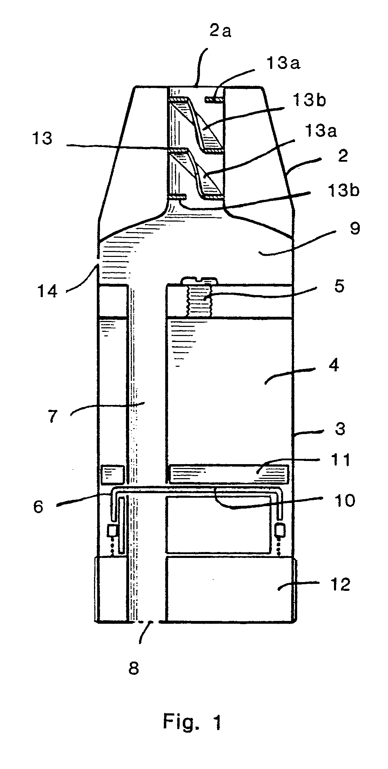

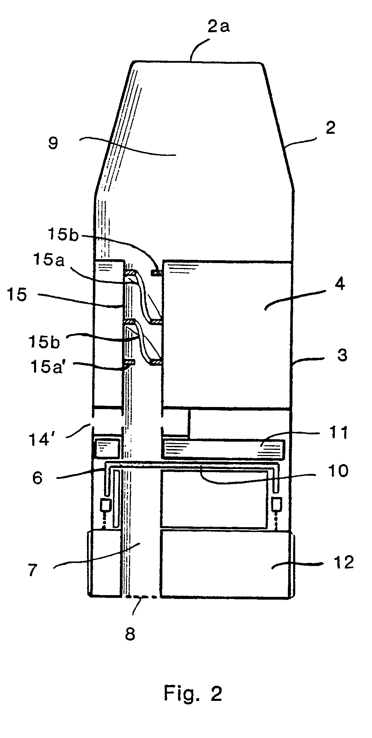

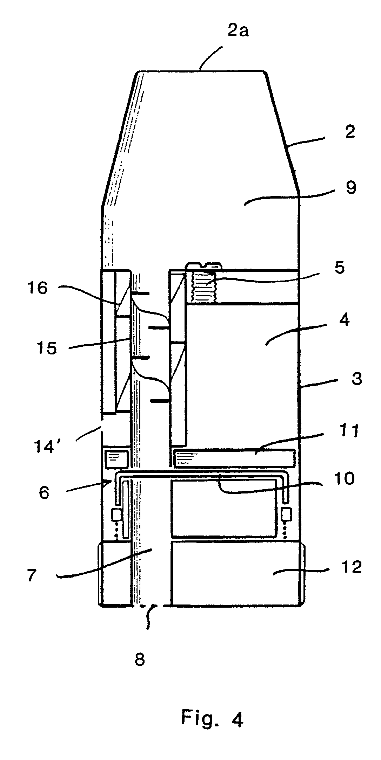

The powder inhalators illustrated in Figures 1-5 are all of a known type (see US-A-4524769). named dosage inhalators, and comprise a nozzle unit 2 with a nozzle aperture 2a located at the top and a container unit 3 with a storage chamber 4 for the active compound (which can be refilled through an upper opening sealed by a plug 5 - shown in Figures 1, 4 and 5 only) and a dosing unit 6 for delivering a dose of the active compound to an air conduit 7. This air conduit 7 extends from a lower air inlet 8, past the dosing unit 6, where the active compound is emitted to the air flow generated by inhalation, and ends in the lower part of the nozzle unit 2, which consists of a free internal chamber 9 (in the embodiments according to Figures 2 and 4 the chamber 9 occupies the whole space inside the nozzle unit 2).

The likewise known dosing unit 6 comprises essentially a perforated membrane 10 in the form of a plane, rotary membrane whose perforations in connection with the storage chamber 4 are filled with powder substance by means of resilient scrapers 11 and which in the area of the air conduit 7 emits the powder substance under the action of the air flow generated by inhalation passing through the perforations of the membrane. The dosing unit 6 is operated by an outer, somewhat knurled grip collar 12, which is connected to the rotary membrane 10 so as to transmit the rotary movement. Thus the dosing is achieved by rotating the membrane 10 a fixed distance by means of the grip collar 12.

According to the present invention deflector devices are arranged in the nozzle unit 2 and/or the container unit 3 and are adapted to powerfully deflect the powder-saturated air flow generated by inhalation. In the illustrated embodiments the deflector devices comprise helical channel portions which give the air flow a rotating, helical pattern of motion. The deflector devices are intended to disrupt aggregated particles by means of the centrifugal force generated when the inhalation air flows through the region of the deflector devices. As discussed above, an effective grinding is accomplished, partly by the particles impacting on the deflecting wall surfaces, partly by mutual collisions between the particles. To attain a sufficient rotary motion of the air flow, it is essential that the radial extension of the hollow space in the cross-section area of the nozzle unit 2 is small compared to the radial extension of the deflector devices. Furthermore, the air flow is additionally accelerated by the fact of the nozzle unit 2 being shaped with a constricted cross-section in the region of the deflector devices.

According to Figure 1 a helical channel portion 13 as described above is arranged at the top of the nozzle unit 2 adjacent the nozzle aperture 2a. The helical channel portion 13, which can be arranged in a detachable liner body in the nozzle unit 2, comprises two interacting helical channel walls 13a and 13b, mutually displaced half a revolution. Furthermore, along the centre line of the helical channel walls there is formed a small, straight hollow space which reduces the flow resistance, at least initially, but which only conducts a small part of the total flow. Consequently air is aspirated into the air intake 8 and the air flow entrains the substance particles in the dozing unit 6, whereupon the particle-saturated air flow enters the chamber 9 of the nozzle unit, where it is mixed with dilution air aspirated through one or more separate air inlets 14 in the side walls of the nozzle unit 2 close to the top end of the container unit 3. Subsequently the composite, particle-saturated air flow is constrained to follow a helical path along the respective helical channel walls 13a, 13b until it leaves the nozzle aperture 2a.

In the embodiment according to Figure 2 the flow proceeds quite freely in the internal chamber 9 of the nozzle unit 2, but the air flow has already been set in helical rotation in the upper part of the air conduit 7 by passing through a similar helical channel portion 15 with helical channel walls 15a, 15b mutually displaced half a revolution. In this case dilution air is aspirated through one or more air inlets 14' located in the central part of the container unit 3 somewhat above the dosing unit 6, that is the dilution should take place before the air flow is set in powerful rotation. The rotary movement will continue as a turbulent flow for at least a short distance up the nozzle unit 2 before the air flow leaves the aperture 2a.

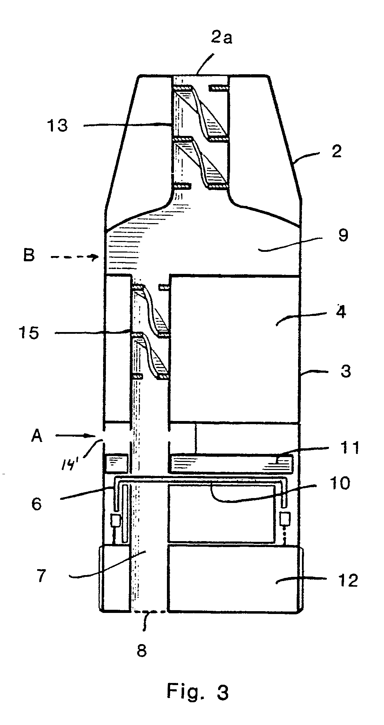

The embodiment according to Figure 3 constitutes essentially a combination of Figures 1 and 2, that is, a helical portion 13 or 15 is arranged both in the upper part of the nozzle unit and in the upper part of the air conduit 7, said dilution air inlets being located either as in Figure 1 (broken arrow B) or as in Figure 2 (arrow A). The directions of rotation according to Figure 3 are the same in both helical channel portions 13, 15, and consequently they cooperate with each other to achieve a higher rotary speed. In principle it is possible to join the two portions together to form a long continuous helical channel portion. As an alternative, it is possible for the two helical channel portions 13, 15 to have opposite directions of rotation, whereby one deliberately aims to generate a turbulent flow in the chamber 9, with the air flow reversing its direction of rotation. Such turbulent flow can also accomplish effective disintegration of aggregates by frequent mutual collisions.

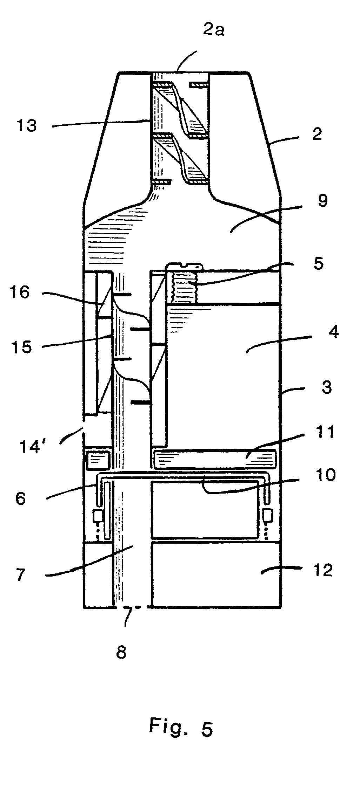

In the embodiments according to Figure 4 and 5 the dilution air is aspirated from an air inlet 14' (located somewhat above the dosing unit 6, as in Figure 2) via an envelope surface 16 with helical channel walls surrounding the upper part of the air conduit 7. Consequently the particle-saturated air flow and the dilution air flow enter the chamber 9 separately, although both air streams are set in rotation (in the same or opposite directions). In the embodiment according to Figure 5 an additional rotary motion is achieved in the helical channel portion 13 of the nozzle unit 2. In principle it is conceivable to omit the helical channel portion 15 in the upper part of the air conduit 7 in the embodiments according to Figures 4 and 5, in which case the rotary motion of the dilution air will act on the particle-saturated air flow in the chamber 9. It is also possible to have the helical channel portion in the envelope surface 16 and in the upper part of the air conduit 7.

Besides the above-mentioned effects of the collisions of the particles and aggregates with the channel walls and with other particles and aggregates, the different helical channel portions 13, 15, 16 (in Figures 1-5) create a constricting effect which increases the flow velocity and thereby further promotes disintegration. However, the constricting effect, that is, the cross-section area of the helical channel portions, must be adjusted to avoid making the inhalation resistance too great for patients with disease of the respiratory tract, such as asthmatics. In general the total cross-section area can be varied between 5 and 50 mm$^2$, while the overall length of the helical channel portions can amount to 5-50 mm.

The ratio between the radial extension of the deflector devices and the radial extension of the central hollow space is an essential factor for the efficient disintegration of powder aggregate into particles in the respirable range, that is, the cross-section area of said hollow space must be adjusted to avoid too much of the particle-saturated air flow to pass through the hollow space and cause an inefficient disintegration. Consequently, it is possible to omit the central hollow space to further promote disintegration. The illustrated embodiments show deflector devices in which the radial extension of the hollow space is about 10-20 % of the radial extension of the deflector devices, as appears from Figures 1-5. In a cross-section in the region of the deflector devices the ratio of the radial extension of the central hollow space to that of the deflector devices can be varied essentially between 0-50 % to attain efficient disintegration and also to provide an inhalation resistance which is not too great for patients suffering from disease of the respiratory tract. In the embodiments according to Figures 1, 3 and 5 the constricted cross-section in the nozzle unit 2 causes an increased acceleration of the particle-saturated air flow and hence a more efficient disintegration.

The invention is applicable in many ways within the scope of the claims. Thus the deflector devices can be placed in different types of powder inhalators (according to the preamble of claim 1), and further the deflector devices can be designed in other ways than in the embodiments.

The channel walls do not have to form continuous guiding surfaces but can consist of a plurality of consecutively arranged, complementary guiding surfaces in the shape of plates or bodies with slightly curved surfaces which together accomplish a powerful deflection of the air flow.

## Claims

1. A powder inhalator for inhalation of an airflow having a container unit (3) containing a pharmacologically active compound in atomized or micronized form, a conduit (7) extending through the container unit (3) and having an air inlet (8), a dosing unit (6) for delivering a dose of the compound to the conduit (7), said airflow being generated by inhalation and being at least partly aspirated through said conduit (7), and a nozzle unit (2) having a nozzle aperture (2a) and communicating with the conduit outlet, means (13, 15, 16) being provided in said inhalator for disintegrating said micronized or atomized compound into particles of a size being in the respirable range, **characterized** in that said disintegrating means (13, 15, 16) comprise deflector devices (13a,13b, 15a,15b, 16) stationarily arranged in the interior of said conduit (7) and/or of said nozzle unit (2), said deflector devices (13a,13b,15a,15b, 16) having a radial extent towards the centerline or longitudinal axis of the conduit (7) respectively the nozzle unit (2), said airflow being accelerated by a constricted cross-section in the region of the deflector devices, said deflector devices (13a, 13b, 15a, 15b, 16) further having a radial extent sufficient to and being oriented such as to impart a rotary movement to said airflow, said deflector devices (13a, 13b, 15a, 15b, 16) thus imparting a deflecting movement to said airflow by accelerating the airflow centripetally by means of said deflector devices (13a, 13b, 15a, 15b, 16), so that particles or aggregates of the compound are ground into smaller particles by colliding with each other and/or impacting on the walls of said deflector devices.

2. Powder inhalator according to claim 1, **characterized** in that a through hole is arranged along the centre axis of said deflector devices (13, 15, 16) to lower the flow resistance.

3. Powder inhalator according to claim 2, **characterized** in that the ratio of the radial extension of said hole to that of said deflector devices is 0 - 50%.

4. Powder inhalator according to claim 1, **characterized** in that at least one additional air inlet (14, 14') is arranged in the container (3) or the nozzle unit (2) for aspiration of dilution air into the airflow generated by inhalation between said dosing unit (6) and said nozzle aperture (2a), and that said deflector devices (13, 15, 16) are arranged to deflect said dilution airflow and/or the the particle-saturated airflow in the region of said air conduit (7).

5. Powder inhalator according to any one of claims 1 - 4, **characterized** in that said deflector devices comprise at least one essentially helically formed channel portion (13, 15, 16).

6. Powder inhalator according to claim 5, **characterized** in that at least one helical channel portion (13), connected to the nozzle aperture (2a), is arranged in said nozzle unit (2).

7. Powder inhalator according to claim 7, **characterized** in that the nozzle unit (2) is shaped with a continuously reducing cross-section area towards the nozzle aperture (2a).

8. Powder inhalator according to claim 5 or 6, **characterized** in that at least one helical channel portion connected to the nozzle unit (2) is arranged in the region of said air conduit (7).

9. Powder inhalator according to claim 6 or 7, **characterized** in that at least the part of the nozzle unit (2) adjacent the container unit (3) consists of a free, internal chamber (9) through which the airflow generated by inhalation passes.

10. Powder inhalator according to any one of the preceding claims in combination with claim 4 in which said additional air inlet (14') is located in the container unit (3) and **characterized** in that said additional air inlet (14') is connected to a separate helical channel (16) which ends in said air conduit (7) or said nozzle unit (2).

11. Powder inhalator according to claim 9, **characterized** in that said separate helical channel portion (16) is arranged in an envelope surface which surrounds said air conduit (7).

12. Powder inhalator according to any one of claims 5 - 10, **characterized** in that the cross-section area of the respective helical channel portion (13, 15, 16) is 5 - 50 mm$^2$ and that the total length of said helical channel portions is 5 - 50 mm.

**Revendications**

1. Un inhalateur de poudre pour l'inhalation d'un écoulement d'air, comprenant un ensemble formant réservoir (3), contenant un composé pharmacologiquement actif, sous forme pulvérisée ou micronisée, une conduite (7) s'étendant à travers de l'ensemble formant réservoir (3) et comprenant une entrée d'air (8), une unité de dosage (6) pour délivrer une dose de mélange à la conduite (7), ledit écoulement d'air étant produit par inhalation et étant, au moins partiellement, aspiré à travers ladite conduite (7), et un ensemble de buse (2), pourvu d'une ouverture de buse (2a) et communiquant avec la sortie de la conduite, des moyens (13, 15, 16) étant prévus dans ledit inhalateur pour désintégrer ledit composé pulvérisé ou micronisé en particules d'une dimension comprise dans la gamme inhalable, caractérisé en ce que lesdits moyens de désintégration (13, 15, 16) comprennent des dispositifs formant déflecteur (13a, 13b, 15a, 15b, 16) montés de façon fixe à l'intérieur de ladite conduite (7) et/ou dudit ensemble de buse (2), lesdits dispositifs formant déflecteur (13a, 13b, 15a, 15b, 16) s'étendant radialement vers l'axe central ou l'axe longitudinal, respectivement, de la conduite (7) et de l'ensemble de buse (2), ledit écoulement d'air étant accéléré par un étranglement en coupe transversale dans la région des dispositifs formant déflecteur, lesdits dispositifs formant déflecteur (13a, 13b, 15a, 15b, 16) ayant en outre une extension radiale suffisante et étant orientés de manière à imprimer un mouvement de rotation audit écoulement d'air, lesdits dispositifs formant déflecteur (13a, 13b, 15a, 15b, 16) imprimant ainsi un mouvement de déviation audit écoulement d'air par une accélération centripète de l'écoulement d'air au moyen desdits dispositifs formant déflecteur (13a, 13b, 15a, 15b , 16), de manière que les particules ou les grumeaux agglomérés de composé soient broyés en fines particules par collision réciproque et/ou par un impact sur les parois desdits dispositifs formant déflecteur.

2. Inhalateur de poudre selon la revendication 1, caractérisé en ce qu'un trou traversant est agencé le long de l'axe central desdits dispositifs formant déflecteur (13, 15, 16) afin d'abaisser la résistance à l'écoulement.

3. Inhalateur de poudre selon la revendication 2, caractérisé en ce que le rapport de l'extension radiale dudit trou sur celle desdits dispositifs formant déflecteur est de 0 à 50 %.

4. Inhalateur de poudre selon la revendication 1, caractérisé en ce qu'au moins une entrée d'air supplémentaire (14, 14') est ménagée dans le réservoir (3) ou dans l'ensemble de buse (2) pour aspirer de l'air de dilution dans l'écoulement d'air produit par inhalation entre ladite unité de dosage (6) et ladite ouverture de buse (2a), et que lesdits dispositifs formant déflecteur (13, 15, 16) sont disposés de manière à dévier ledit écoulement d'air de dilution et/ou l'écoulement d'air saturé de particules dans la région de ladite conduite d'air (7).

5. Inhalateur de poudre selon l'une quelconque des revendications 1 à 4, caractérisé en ce que lesdits dispositifs formant déflecteur comprennent au moins une partie de canal essentiellement de forme hélicoïdale (13, 15, 16).

**6.** Inhalateur de poudre selon la revendication 5, caractérisé en ce qu'au moins une partie formant canal hélicoïdal (13) reliée à l'ouverture de buse (2a) est agencée dans ledit ensemble de buse (2).

**7.** Inhalateur de poudre selon la revendication 7, caractérisé en ce que l'ensemble de buse (2) est conformé de manière que sa surface en coupe transversale se réduise progressivement vers l'ouverture de buse (2a).

**8.** Inhalateur de poudre selon la revendication 5 ou 6, caractérisé en ce qu'au moins une partie formant canal hélicoïdal, reliée à l'ensemble de buse (2), est agencée dans la région de ladite conduite d'air (7).

**9.** Inhalateur de poudre selon la revendication 6 ou 7, caractérisé en ce qu'au moins la partie de l'ensemble de buse (2) adjacente à l'ensemble formant réservoir (3) est constituée par une chambre intérieure libre (9) traversée par l'écoulement d'air produit par l'inhalation.

**10.** Inhalateur de poudre selon l'une quelconque des précédentes revendications, en combinaison avec la revendication 4, dans lequel ladite entrée d'air supplémentaire (14') est située dans l'ensemble formant réservoir (3) et caractérisé en ce que ladite entrée d'air supplémentaire (14') est reliée à un canal hélicoïdal séparé (16) qui aboutit dans ladite conduite d'air (7) ou ledit ensemble de buse (2).

**11.** Inhalateur de poudre selon la revendication 9, caractérisé en ce que ladite partie formant canal hélicoïdal séparé (16) est agencée dans une surface formant enveloppe qui entoure ladite conduite d'air (7).

**12.** Inhalateur de poudre selon l'une quelconque des revendications 5 à 10, caractérisé en ce que la surface en coupe transversale de la partie respective en forme de canal hélicoïdal (13, 15, 16) est de 5 à 50 mm² et que la longueur totale desdites parties formant canal hélicoïdal est de 5 à 50 mm.

## Patentansprüche

**1.** Pulverinhalator zur Inhalation eines Luftstroms mit einer eine pharmakologisch wirksame Verbindung in zerstäubter oder mikronisierter Form enthaltenden Behältereinheit (3), einer durch die Behältereinheit (3) verlaufenden Leitung (7), die einen Lufteinlaß (8) aufweist, einer Dosierungseinheit (6) zur Abgabe einer Dosis der Verbindung an die Leitung (7), wobei der Luftstrom durch Inhalation erzeugt und zumindest teilweise durch die Leitung (7) angesaugt wird, und einer Düseneinheit (2), die eine Düsenöffnung (2a) aufweist und mit dem Leitungsauslaß in Verbindung steht, wobei im Inhalator Mittel (13, 15, 16) zur Zerteilung der mikronisierten oder zerstäubten Verbindung zu Teilchen einer im einatembaren Bereich liegenden Größe vorgesehen sind, dadurch gekennzeichnet, daß diese Zerteilungsmittel (13, 15, 16) Ablenkungseinrichtungen (13a,13b, 15a,15b, 16) umfassen, die ortsfest im Inneren der Leitung (7) und/oder der Düseneinheit (2) angeordnet sind, wobei die Ablenkungseinrichtungen (13a, 13b, 15a,15b, 16) zur Mittellinie oder Längsachse der Leitung (7) bzw. der Düseneinheit (2) hin eine radiale Erstreckung aufweisen, wobei der Luftstrom durch einen verengten Querschnitt im Bereich der Ablenkungseinrichtungen beschleunigt wird, und wobei die Ablenkungseinrichtungen (13a, 13b, 15a,15b, 16) weiters eine radiale Erstreckung aufweisen, die ausreicht und so gerichtet ist, daß sie den Luftstrom in eine Drehbewegung versetzt, wodurch die Ablenkungseinrichtungen (13a,13b, 15a,15b, 16) den Luftstrom in eine Ablenkungsbewegung versetzen, indem sie den Luftstrom durch die Ablenkungseinrichtungen (13a,13b, 15a,15b, 16) zentripetal beschleunigen, so daß Teilchen oder Aggregate der Verbindung durch gegenseitiges Zusammenstoßen oder Auftreffen auf die Wände der Ablenkungseinrichtungen zu kleineren Teilchen zerrieben werden.

**2.** Pulverinhalator nach Anspruch 1, dadurch gekennzeichnet, daß ein durchgehendes Loch entlang der Mittelachse der Ablenkungseinrichtungen (13, 15, 16) zur Herabsetzung des Strömungswiderstandes angeordnet ist.

**3.** Pulverinhalator nach Anspruch 2, dadurch gekennzeichnet, daß das Verhältnis der radialen Erstreckung dieses Loches zu jener der Ablenkungseinrichtungen 0-50% ist.

**4.** Pulverinhalator nach Anspruch 1, dadurch gekennzeichnet, daß zumindest ein zusätzlicher Lufteinlaß (14, 14') in der Behälter- (3) oder der Düseneinheit (2) zum Ansaugen von Verdünnungsluft in den durch Inhalation zwischen der Dosierungseinheit (6) und der Düsenöffnung (2a) erzeugten Luftstrom angeordnet ist, und daß die Ablenkungseinrichtungen (13, 15, 16) zur Ablenkung des Verdünnungsluftstroms und/oder des mit Teilchen gesättigten Luftstroms im Bereich der Luftleitung (7) angeordnet sind.

5. Pulverinhalator nach einem der Ansprüche 1 bis 4, dadurch gekennzeichnet, daß die Ablenkungseinrichtungen zumindest einen im wesentlichen schraubenförmigen Kanalabschnitt (13, 15, 16) umfassen.

6. Pulverinhalator nach Anspruch 5, dadurch gekennzeichnet, daß zumindest ein mit der Düsenöffnung (2a) verbundener schraubenförmiger Kanalabschnitt (13) in der Düseneinheit (2) angeordnet ist.

7. Pulverinhalator nach Anspruch 6, dadurch gekennzeichnet, daß die Düseneinheit (2) zur Düsenöffnung (2a) hin mit einer sich kontinuierlich verkleinernden Querschnittsfläche geformt ist.

8. Pulverinhalator nach Anspruch 5 oder 6, dadurch gekennzeichnet, daß zumindest ein schraubenförmiger, mit der Düseneinheit (2) verbundener Kanalabschnitt im Bereich der Luftleitung (7) angeordnet ist.

9. Pulverinhalator nach Anspruch 6 oder 7, dadurch gekennzeichnet, daß zumindest der an die Behältereinheit (3) angrenzende Teil der Düseneinheit (2) aus einer freien Innenkammer (9) besteht, durch welche der durch Inhalation erzeugte Luftstrom hindurchgeht.

10. Pulverinhalator nach einem der vorhergehenden Ansprüche in Kombination mit Anspruch 4, bei welchem sich der zusätzliche Lufteinlaß (14') in der Behältereinheit (3) befindet und welcher dadurch gekennzeichnet ist, daß der zusätzliche Lufteinlaß (14') mit einem separaten schraubenförmigen Kanal (16) verbunden ist, der in der Luftleitung (7) oder der Düseneinheit (2) endet.

11. Pulverinhalator nach Anspruch 9, dadurch gekennzeichnet, daß der separate schraubenförmige Kanalabschnitt (16) in einer die Luftleitung (7) umgebenden Umhüllungsfläche angeordnet ist.

12. Pulverinhalator nach einem der Ansprüche 5-10, dadurch gekennzeichnet, daß die Querschnittsfläche des jeweiligen schraubenförmigen Kanalabschnitts (13, 15, 16) 5-50 mm$^2$ beträgt und daß die Gesamtlänge der schraubenförmigen Kanalabschnitte 5-50 mm beträgt.

Fig. 1

Fig. 2

Fig. 3

Fig. 4

Fig. 5